# EUROPEAN PATENT APPLICATION

(11) **EP 1 184 462 A2**
(43) Date of publication of application: **06.03.2002**
(21) Application number: 01203275.1
(22) Date of filing: 31.08.2001
(51) Int. Cl.: C12N 15/70, C12N 15/74, C12Q 1/18

(54) **Identification of targets of antimicrobial compounds**

(30) Priority: 01.09.2000 US 229965 P
(71) Applicant: SmithKline Beecham Corporation, Philadelphia, PA 19103 (US); SMITHKLINE BEECHAM PLC, Brentford, Middlesex TW8 9EP (GB)
(72) Inventor: Fan, Frank, GlaxoSmithKline, Collegeville, Pennsylvania 19426 (US); He, Yiping, GlaxoSmithKline, Collegeville, Pennsylvania 19426 (US); Huang, Jianzhong, GlaxoSmithKline, Collegeville, Pennsylvania 19426 (US); Jiang, Xinhe, GlaxoSmithKline, Collegeville, Pennsylvania 19426 (US); McDevitt, Damien, GlaxoSmithKline, Collegeville, Pennsylvania 19426 (US); Rosenberg, Martin, GlaxoSmithKline, Collegeville, Pennsylvania 19426 (US); St John, Annemarie, GlaxoSmithKline, King of Prussia, Pennsylvania 19406 (US)
(74) Representative: Connell, Anthony Christopher

(57) **Abstract**

A method of determining the cellular or genetic target of an antimicrobial compound includes cloning of a bacterial gene into an expression vector with an inducible promoter and determining whether increasing expression of the cloned gene in the cell will result in resistance to an antimicrobial compound. Also, the method can include incorporation of every gene of a bacterial strain into expression vectors having an inducible promoter, induction, treating with an antimicrobial compound, isolating the gene clone that confers cells resistant to the compound, and determining the identity of the resistant gene by various methods including DNA microarrays and gene sequencing.

PCTSEQLIST

SEQUENCE LISTING

<110> SMITHKLINE BEECHAM CORPORATION
      SMITHKLINE BEECHAM p.l.c.

<120> IDENTIFICATION OF TARGETS OF
  ANTIMICROBIAL COMPOUNDS

<130> P51167

<140> TO BE ASSIGNED
<141> 2001-08-31

<150> 60/229,965
<151> 2000-09-01

<160> 11

<170> FastSEQ for Windows Version 4.0

<210> 1
<211> 6
<212> DNA
<213> Staphylococcus aureus

<400> 1
aggagg
6

<210> 2
<211> 5
<212> DNA
<213> Staphylococcus aureus

<400> 2
aaact
5

<210> 3
<211> 10
<212> DNA
<213> Staphylococcus aureus

<400> 3
ggcgcgccaa
10

<210> 4
<211> 6
<212> DNA
<213> Staphylococcus aureus

PCTSEQLIST

<400> 4
aaacta
6

<210> 5
<211> 10
<212> DNA
<213> Staphylococcus aureus

<400> 5
ttggcgcgcc
10

<210> 6
<211> 25
<212> DNA
<213> Staphylococcus aureus

<400> 6
aaactatgtt aaatcttgaa aacaa
25

<210> 7
<211> 29
<212> DNA
<213> Staphylococcus aureus

<400> 7
ttggcgcgcc gattattata aaagcaaaa
29

<210> 8
<211> 24
<212> DNA
<213> Staphylococcus aureus

<400> 8
aaactatgaa cgtgggtatt aaag
24

<210> 9
<211> 27
<212> DNA
<213> Staphylococcus aureus

<400> 9
ttggcgcgcc tattttcccc attttat
27

<210> 10
<211> 24

PCTSEQLIST
<212> DNA
<213> Staphylococcus aureus

<400> 10
aaacatgtta gaatttgaac aagg
24

<210> 11
<211> 28
<212> DNA
<213> Staphylococcus aureus

<400> 11
ttggcgcgcc gattaacgtc ttgttctt
28

## Description

### FIELD OF THE INVENTION

The present invention relates to the screening of antimicrobial compounds and to the bacterial cells and related molecules which are targets of antimicrobial compounds. More particularly, it pertains to methods and assays for identifying the targets of antimicrobial compounds and the mechanisms of their action.

### BACKGROUND OF THE INVENTION

Many chemical compounds exhibit antimicrobial activity, but through unknown mechanisms. Currently, the technology for identification of the cellular or genetic targets of these compounds is limited. In one instance, a genetic approach can be used to identify the target of a drug. This involves isolation of mutants that are resistant to the drug, identification of the resistant determinants, and biochemical characterization of the resistant determinants. Identification of the resistant determinant is accomplished by making a genomic library with the DNA fragments from the resistant mutant and then transferring the library into the sensitive strain to select strains which have become resistant to the drug. Then, the DNA fragment on the plasmid, which confers the resistance to the sensitive strain, can be extracted from the resistant strain and sequenced to find out what gene or genes are involved.

Additionally, a biochemical approach can be used to help understand the mechanism of drug action and/or identify which physiological process is affected by the drug. For example, the macromolecule biosynthesis that is affected by the drug can be examined. Finally, genomics/DNA microarrays and proteomics have recently been developed to explore drug-induced alterations in gene expression, which can provide insight into the mechanism of action of drugs.

In view of the increasing prevalence of new bacterial strains resistant to known antimicrobial compounds, there is a need for an additional method of identifying the targets and action of antimicrobial compounds. This information can aid in the design and development of novel antimicrobial compounds to combat the resistance of bacterial strains to known antimicrobial compounds.

### SUMMARY OF THE INVENTION

The present invention is directed to a method of identifying the targets and action of antimicrobial compounds using prokaryotic (herein also "bacterial") and fungal genes and/or gene expression libraries of various organisms. The expression of those genes is under control of an inducible promoter system and a strong ribosome binding site (herein "RBS") from an expression vector. In such a method, bacterial cells carrying the library of genes on the expression vector are treated with antimicrobial compounds both in the presence and absence of an inducer. The cells and only those cells that over-expressed the target gene of an antimicrobial compound from the expression vector will show more resistance to the compound. Thus, the target can be identified through the identification of resistant cells carrying the target gene on plasmids and isolation and characterization of plasmid clones carrying the target gene.

In one embodiment, the present invention comprises a method of inducing expression of certain genes that are the targets of known antimicrobial compounds and showing that cells carrying these target genes (herein also "clones") are more resistant to the antimicrobial compounds when expression of the target genes is induced.

In another embodiment, the present invention comprises a method of constructing a gene expression library of a prokaryotic organism (herein also "bacterial strain") in which every open reading frame (herein "ORF") of the genome from the organism may be cloned into an expression vector under control of an inducible promoter system. The method further comprises methods to identify resistant clones from the library and therefore the targets of antimicrobial compounds.

In yet another embodiment, the present invention comprises a method of constructing an expression vector (herein also "plasmid"), which includes an inducible promoter system, a strong RBS, and multiple cloning sites into which intact ORFs of a prokaryotic organism can be cloned.

It is to be understood that both the foregoing general description and the following detailed description are exemplary, but are not restrictive, of the invention.

### BRIEF DESCRIPTION OF THE DRAWINGS

The invention is best understood from the following detailed description when read in connection with the accompanying drawings, in which:

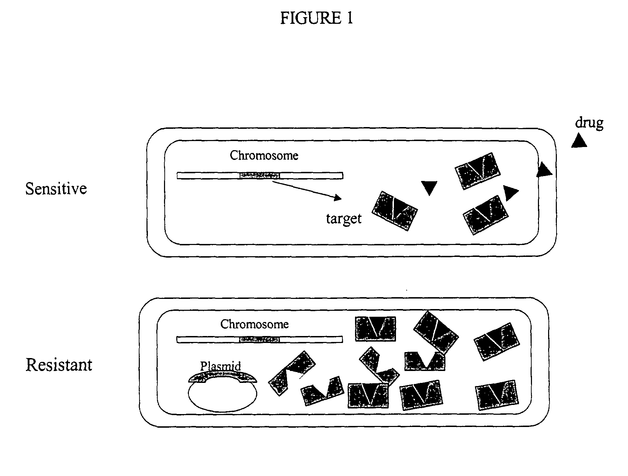

Figure 1 shows a sensitive bacterium where a target binds its drug, and the amount of drug is in excess (upper image). This figure also shows a resistant bacterium where a target binds its drug, and the amount of drug is in limiting, not affecting all or substantially all target molecules (lower image). Each type of bacteria is described and exemplified in more detail herein.

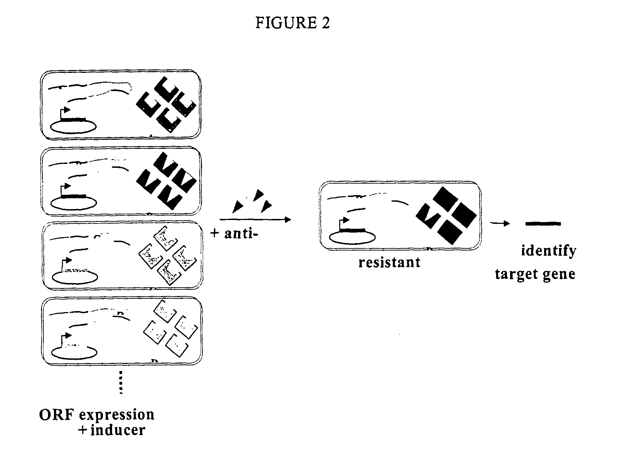

Figure 2 shows a schematic of a selection of resistant clones and a target gene as described and exemplified in more detail herein. Each schematic target binds a different molecule, as shown by the different shaped binding sites, being triangular, square, semicircular, and oblate. In this example, the target with the triangular binding site is selected by the triangular molecule. The target gene thereby selected is cloned out of the bacteria.

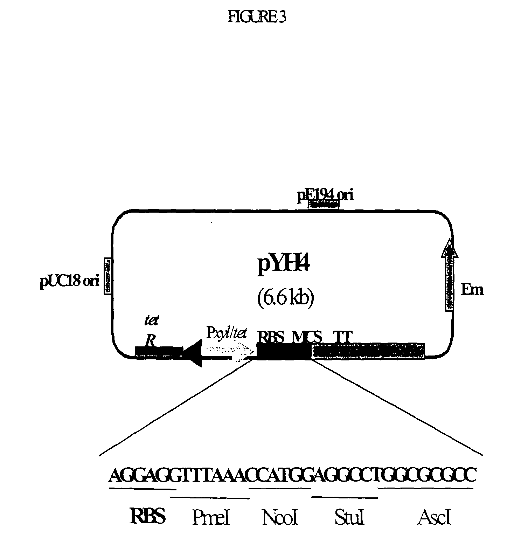

Figure 3 shows an exemplary expression vector with an inducible promoter system as used in the methods of the present invention.

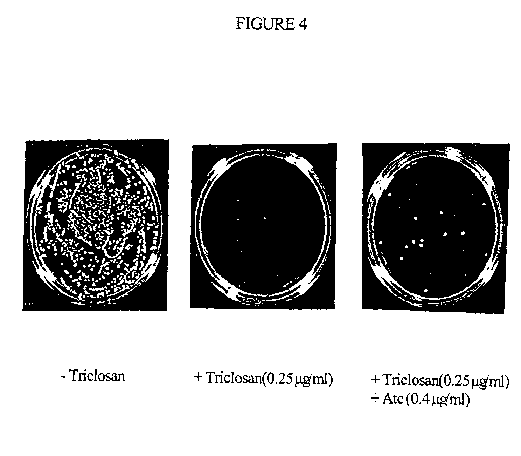

Figure 4 shows selection of triclosan resistant colonies on TSA plates from a mixed culture containing fabI and non-fabI clones, (1, left) without an inducer or an antimicrobial compound, (2, center) in the presence of only the triclosan antimicrobial compound, and (3, right) in the presence of triclosan and an inducer.

### DETAILED DESCRIPTION OF THE INVENTION

It has been demonstrated that the resistance of a bacterial strain to an antimicrobial compound can be conferred by increasing amounts of the bacterial target in the cell. More specifically, if (1) a bacterial gene is cloned into an expression vector, (2) its corresponding protein is induced to be expressed at high levels, and (3) it is a target for an antimicrobial compound, the increasingly expressed protein will confer on the cell more resistance to the antimicrobial compound. This resistance can enable the detection of the target of an antimicrobial compound. As increasing amounts of the target protein are produced, the antimicrobial compound (herein also "drug") is insufficient to inhibit/inactivate all of the target proteins; the remaining proteins are sufficient to maintain the cellular, biochemical, and physiological functions of the cell; and the cell survives and proliferates.

In addition, this concept can be expanded to include every open reading frame of a genome for identification of the target(s) of any antimicrobial compound. Specifically, an inducible expression library containing all of the open reading frames of a bacterial strain or other prokaryotic organism can be constructed. Because each ORF is cloned into an expression vector, over-expression of each ORF can be achieved. Preferably, the expression vector is a shuttle vector which allows replication in *E. coli* and transformation into the host organism, such as *S. aureus*. When the cells containing the library are treated with the antibacterial compound, the cell with the target ORF will be more resistant to the compound and can be identified by the methods described in the present invention. Therefore, the methods of the present invention should enable identification of the target(s) of any antimicrobial compound.

"Organism" for purposes of the present invention is defined as (i) a prokaryote, including but not limited to, a member of the genus *Streptococcus, Staphylococcus, Bordetella, Corynebacterium, Mycobacterium, Neisseria, Haemophilus, Actinomycetes, Streptomycetes, Nocardia, Enterobacter, Yersinia, Fancisella, Pasturella, Moraxella, Acinetobacter, Erysipelothrix, Branhamella, Actinobacillus, Streptobacillus, Listeria, alymmatobacterium, Brucella, Bacillus, Clostridium, Treponema, Escherichia, Salmonella, Kleibsiella, Vibrio, Proteus, Erwinia, Borrelia, Leptospira, Spirillum, Campylobacter, Shigella, Legionella, Pseudomonas, Aeromonas, Rickettsia, Chlamydia, Borrelia* and *Mycoplasma*, and further including, but not limited to, a member of the species or group, *Group A Streptococcus, Group B Streptococcus, Group C Streptococcus, Group D Streptococcus, Group G Streptococcus, Streptococcus pneumoniae, Streptococcus pyogenes, Streptococcus agalactiae, Streptococcus faecalis, Streptococcus faecium, Streptococcus durans, Neisseria gonorrheae, Neisseria meningitidis, Staphylococcus aureus, Staphylococcus epidermidis, Corynebacterium diptheriae, Gardnerella vaginalis, Mycobacterium tuberculosis, Mycobacterium bovis, Mycobacterium ulcerans, Mycobacterium leprae, Actinomyctes israelii, Listeria monocytogenes, Bordetella pertusis, Bordatella parapertusis, Bordetella bronchiseptica, Escherichia coli, Shigella dysenteriae, Haemophilus influenzae, Haemophilus aegyptius, Haemophilus parainfluenzae, Haemophilus ducreyi, Bordetella, Salmonella typhi, Citrobacter freundii, Proteus mirabilis, Proteus vulgaris, Yersinia pestis, Kleibsiella pneumoniae, Serratia marcessens, Serratia liquefaciens, Vibrio cholera, Shigella dysenterii, Shigella flexneri, Pseudomonas aeruginosa, Franscisella tularensis, Brucella abortis, Bacillus anthracis, Bacillus cereus, Clostridium perfringens, Clostridium tetani, Clostridium botulinum, Treponema pallidum,* *Rickettsia rickettsii* and *Chlamydia trachomitis*; (ii) an archaeon, including but not limited to Archaebacter; and (iii) a unicellular or filamentous eukaryote, including but not limited to, a protozoan, a fungus, a member of the genus *Saccharomyces, Kluveromyces*, or *Candida,* and a member of the species *Saccharomyces ceriviseae, Kluveromyces lactis*, or *Candida albicans*. In particularly preferred embodiments of the present invention, *Staphylococcus aureus* (*S. aureus*) is the organism tested.

In its most general form, the present invention comprises a method of using prokaryotic ORFs/genes engineered into expression vectors under control of an inducible promoter to determine the targets of antimicrobial compounds. Individual bacterial ORFs/genes of an organism/strain may be incorporated into expression vectors to form a library that comprises substantially all of the open reading frames of a bacterial strain.

### Construction of an expression vector

The methods of the present invention use an expression vector, such as the pYH4 vector shown in Figure 3, containing a Pxyl/tet regulatory system (hybrid transcription promoter and tetR transcription repressor) (details of the Pxyl/tet system are found in Geissendorfer, *et al., Appl. Microbiol. Biotechnol.* 33: 657-663 (1990), a ribosome binding site (labeled "RBS"), such as one having the sequence: 5'-AGGAGG-3'[SEQ ID NO:1], one or more cloning sites, and a transcriptional terminator (labeled "TT"). The spacing between the ribosome binding site and the translation start site in the vector is limited to about 8 base pairs as in other commonly used expression vectors. In addition, that region should be as AT-rich as possible.

The cloning (herein also "restriction") sites in the pYH4 vector are PmeI, NcoI, StuI, and AscI. The PmeI and AscI sites are used for cloning of the ORFs into the vector. The PmeI and AscI sites/enzymes are preferred because these enzymes do not cut along the DNA sequence of most of the ORFs of the *S. aureus* or other strain so that substantially all of the ORFs are left intact when using these sites/enzymes. Further, these sites are not present elsewhere on the pYH4 vector, further facilitating the complete cloning of most of the ORFs of *S. aureus* or other organism into the expression vector.

With regard to the ORFs, every ORF cloned into the vector has an invariable N-terminal "AAACT"[SEQ ID NO:2] sequence stretch immediately upstream of the second position of the initiation codon. Every ORF also has a C-terminal "GGCGCGCCAA"[SEQ ID NO:3] tail. The vector also includes (1) the pE194ori, *S. aureus* plasmid pE194 replicon, allowing replication in *S. aureus*; (2) a pUC18ori, an *E. coli* replicon allowing replication in an *E. coli* host; and an Em (erythromycin) selection marker which confers the vector with resistance to Em (an antimicrobial compound commonly used in cloning vectors to select cells with the vector). The presence of the two replicons allows the vector to act as a shuttle vector such that a gene can be replicated in both hosts (*e.g. E. coli* and *S. aureus,* among other organisms).

The pYH4 vector is also useful for the present invention because of the increased expression it provides when it is induced. To test for this increased expression, a lacZ ORF was cloned into the pYH4 vector with the features described above. Expression of lacZ was measured with the vector in both induced and uninduced forms. In fact, induction of the vector provided greater than a 40-fold increase in LacZ expression. Further, the increase in expression was dependent on the concentration (herein also "dosage") of the inducer.

The expression vector used or any of its features may be modified from those described above in order to be used with different organisms, particularly bacterial strains to accommodate their ORFs. In another embodiment of the present invention, the Pspac/LacI system is inserted into the pYH4 to replace the Pxyl/tet system. The Pspac/LacI system is readily available and known to one of skill in the art. Details of the Pspac system can be found in Vagner, *et al., Microbiology* 144: 3097-3104 (1998), the entire disclosure of which is herein incorporated by reference.

Once the expression vector is constructed, known ORFs/genes from an organism are cloned into the expression vector such that their expression is under the control of the expression system on the vector. These genes are tested for being targets of the known antimicrobials or compounds while in the vector. The vector (herein also "clone") is then induced to express the bacterial gene by addition of a suitable inducer, such as anhydrotetracycline (in another embodiment, tetracycline may be used). The expression of the gene will increase the presence of the corresponding bacterial protein or other cell component. If that protein or component is a target of an antimicrobial compound, its corresponding resistance to the antimicrobial compound will increase and manifest itself by further growth of the bacteria. More particularly, the enhanced growth of specific induced clones (representing individual genes or expression vectors containing the bacterial genes) versus uninduced clones displays the cellular or genetic target of the antimicrobial compound being tested.

Particular genes of *S. aureus* which have been identified as targets include fabI (a fatty acid biosynthesis gene of bacteria) for triclosan; murAl (a bacterial cell wall synthesis gene) for phosphomycin; and metRS (a bacterial tRNA synthetase gene) for metRS inhibitors. The genes incorporated into a vector are analyzed to determine if increasing the expression of such genes results in resistance to the antimicrobial compounds.

### Construction of an inducible ORF expression library

The present invention is also directed to a library formed by cloning substantially all of the open reading frames of a bacterial genome into expression vectors under the control of an inducible promoter. As an example, every open reading frame of *S. aureus* or other organism's genome is amplified by the polymerase chain reaction (herein "PCR") using primers which can be designed based on the sequence of each open reading frame. Once amplified, each gene can be cloned into the expression vector under control of an inducible promoter using known techniques in the art. The resulting library is used to transform *S. aureus* host cells to replicate the genes and increase their copy number and expression. For purposes of the present invention, "substantially all" refers to 95% or more of the genes in the genome.

Once the genes have been cloned into the expression vector to form a library, the library containing the genes of the genome are gridded into microtiter plates such that each well contains one clone or ORF. The library is divided into two sets, one set with the gene in its uninduced form and one set with inducer added such that the gene is in its induced form. The induced form of the gene library expresses increasing amounts of the bacterial targets as compared to the uninduced form.

Both sets of genes (induced and uninduced) are contacted with an antimicrobial agent. In both the presence and absence of the inducer, all cells, except for those cells with the gene encoding the target of the agent, will be killed or greatly inhibited by the antimicrobial agent. The cells with the target gene will show increased resistance to the agent and proliferate.

For example, the genes of an organism are amplified by PCR and purified. The purified PCR gene product is digested with AscI and ligated into the PmeI and AscI-digested pYH4 vector, the features of which are described above. The ligation products (cloned genes) are then transformed into an *E. coli* host such as DH10B for replication and the colonies resistant to erythromycin (Em), *i.e.,* representing genes successfully cloned into vectors, are selected on LB Em plates. These clones can then be transferred into *S. aureus* and tested for targets of an antimicrobial compound.

### Use of Inducer

Tetracycline, anhydrotetracyline, and other known or useful inducers of the Pxyl/tet expression system and other expression systems are used in the methods of the present invention. Nevertheless, tetracycline is toxic to certain cells, which may limit the levels of induction in certain instances. In order to combat its toxicity, if tetracycline is used as the inducer, and where toxic, a tetracycline resistance gene can be inserted directly into the chromosome of the strain of the organism. The tetracycline resistance gene prevents binding of tetracycline to the ribosome and, thus, limits or eliminates the toxicity of the tetracycline to the bacterial cells being expressed.

### Primer design

Primers used for amplification are designed from knowledge of the sequence of the entire genome of the organism. Sequencing can be accomplished by any technique used in the art. Once ORF sequences are identified, primers are produced for both the 5' (herein "forward") and 3' (herein "reverse") ends of the gene and they are selected from sequences flanking the open reading frame of each gene. Each primer is approximately 25 bases in length. It is critical to identify the correct ORFs to be amplified so that the intact gene products can be expressed. In other words, it is necessary to identify the correct translation start site and stop codon for substantially all of the genes in the genome of the organism. The "forward" or "N-terminal" primer comprises a 6 base-pair sequence tail: 5'-AAACTA-3'[SEQ ID NO:4], along with a variable region starting at the second position of the start codon and its immediate downstream sequences. The "reverse" or "C-terminal" primer comprises an invariable tail:
5'-TTGGCGCGCC-3'[SEQ ID NO:5], along with a variable region. This variable region can be either the region containing the stop codon or its downstream region.

### Transfer of the library into an organism

The plasmid DNA from individual clones of the library is transferred into an organism, for example *S. aureus* RN4220 by electroporation using techniques known in the art. The library is gridded into microtiter plates for storage, growth, and later experimentation.

### PCR Amplifications of ORFs and Product Purification

The PCR is used to amplify the genes of the genome. The PCR reaction products are analyzed, preferably by agarose gel electrophoresis to measure the amplification and product specificity, and the amplification product is purified by known techniques in the art.

### Detection of resistant clones and identification of targets

### (a) Population approach

*S. aureus* or other organism containing an ORF expression library under the control of an inducible promoter will be grown on microtiter plates such that each clone/ORF is grown to about similar concentration. The cultures are then pooled and serially diluted. About 10⁵ bacteria from the diluted cultures are spread onto solid medium plates that contain an antimicrobial compound with the presence or absence of an inducer of expression. If a cell carries an ORF clone that is the target of the given compound, induction of the ORF will increase the presence of the target in the cell. Thus the cell with the ORF clone will survive and form a colony on the plate with the compound at the concentration that normally will kill the cell. The ORF clone can then be extracted from the cell and sequenced to determine its identity and therefore identification of the target of the compound.

### (b) Microarray approach

In the microarray approach, a population of *S. aureus* or other organism containing an ORF expression library under the control of an inducible promoter will be grown on microtiter plates such that each clone/ORF is grown to about similar concentration. The cultures are then pooled and divided into two equivalent sub-populations. One pool is treated with an antimicrobial compound and the other pool treated with the same compound but also with an inducer. After a period of growth the plasmid DNA is then extracted from the pools, respectively.

The plasmid DNA is used as templates to prepare probes that are fluorescence tagged or radiolabeled by primer extension methods. Techniques of fluorescence labeling are known in the art, such as, for example, those set forth by Iyer, *et al., Science* 283: 83-87 (1999). The probes from uninduced pool are labeled with a green signal, while the probes from induced pool are labeled with a red signal. The probes from both pools are then mixed and hybridized with DNA microarrays having the ORFs of the genome. Because the target clone of the antimicrobial compound will be enriched due to cells with the target clone are more resistant to the compound, there will be more labeled target DNA in the probes. Therefore the target of the antimicrobial compound will give more hybridization signals on the microarrays and thus allow identification of the target.

Other known techniques of labeling the genes for identifying the target of an antimicrobial compound may be used. In general, when these techniques are used with DNA microarrays, increased strength of the signal from hybridization with the DNA microarrays is indicative of the target gene.

### (c) Individual clone approach

The individual clones of the library will be gridded into microtiter plates and contacted with an antimicrobial compound. Then, cell growth will be compared between and among the induced cells and uninduced cells to confirm that a cell suspected to be resistant is actually a resistant clone. Growth among uninduced cells will be compared because the basal (herein also "uninduced") level of expression may already be able to confer the resistance to the antimicrobial compound.

More specifically, substantially all of the genes in the *S*. *aureus* or other organism's genome are cloned into vectors and are transferred into host cells. The host can be either *S. aureus* or a different species, such as from the organisms listed above. One preferred embodiment includes the use of *S. aureus* as the host. The induced and uninduced individual clones are grown on microtiter plates to which is added an antimicrobial compound at a concentration of about 1-2 times the minimum inhibitory concentration (herein also "MIC"). In the microtiter format, each well represents one clone or ORF.

If addition of the candidate compound results in death of the organism, such as *S. aureus* cells, then it is presumed that the candidate compound is attacking some protein target within the cell. In contrast, the well representing the resistant clone will show growth (this can be detected by increased turbidity of the sample). Once the resistant clone is identified, its gene sequence or ORF can be easily determined from its gridded position on the plate.

### Examples:

The examples below are carried out using standard techniques, which are well and routine to those of skill in the art, except where described in detail. The examples illustrate but do not limit the present invention.

### Example 1 Overepression of S. aureus FabI confers triclosan resistance

The *S. aureus* genome has been sequenced to contain 48 contigs and approximately 2437 genes have been identified from the genome. The following ORFs have been identified from the *S. aureus* genome for PCR amplification and cloning into the expression vector: (1) fabI ORF - encoding 256 amino acid residues for the protein enoyl-acyl carrier protein reductase; (2) fabH ORF - encoding 313 amino acid residues for the protein betaketoacyl-ACP synthase III; and (3) ftsZ ORF - encoding 390 amino acid residues for a protein involved in midcell septum formation in actively dividing bacteria. In addition, the murA1, murA2, def1, def0, metRS, ileRS, fabF, gyrA, and gyrB ORFs have been studied as potential targets of antimicrobial compounds.

The PCR method used is as follows: 50-µl PCR-reactions contained 10 mM Tris-HCl (pH 8.85), 25 mM KCI, 5 mM (NH₄)₂SO₄, 2 mM MgSO₄, 0.2 mM dNTPs, 2.5 units of Pwo DNA polymerase (Boehringer Mannheim), 0.6 µM of up and lower primers, and 0.5 µg of
*S. aureus* WCUH29 chromosomal DNA. After denaturation for 2 minutes at 94°C, 30 thermal cycles comprising 15 seconds at 94°C, 30 seconds at 52°C, and 2 minutes at 72°C, were performed. Primers used for PCR amplification were: for fabI ORF, fabI up (5'-AAACTATGTTAAATCTTGAAAACAA-3')[SEQ ID NO:6] and fabI lower (5'-TTGGCGCGCCGATTATTATAAAAGCAAAA-3')[SEQ ID NO:7]; for fabH ORF, fabH up (5'-AAACTATGAACGTGGGTATTAAAG-3')[SEQ ID NO:8] and fabH lower (5'-TTGGCGCGCCTATTTTCCCCATTTTAT-3')[SEQ ID NO:9]; and for ftsZ ORF, ftsZ up (5'-AAACATGTTAGAATTTGAACAAGG-3')[SEQ ID NO: 10] and fts lower (5'-TTGGCGCGCCGATTAACGTCTTGTTCTT-3')[SEQ ID NO:11]. After PCR amplification, the PCR Products were purified with a QIAquick PCR purification kit, digested with AscI enzyme, and then cloned into the pYH4 expression vector as described above.

The fabI ORF clone was tested for resistance to the FabI-specific antimicrobial compound, triclosan, and the testing revealed that the increased production of the FabI ORF after induction resulted in an 8 to 16-fold increase in the MIC of the triclosan (*i.e.*, the concentration necessary to inhibit the induced bacterial strain). This confirms that FabI is a target of triclosan.

The pYH4 expression vector was used to clone the FabI ORF and it served as a control for the experiment. The data is as follows in Table 1.

**Table 1**

| **MIC concentration (µg/ml) of triclosan necessary to inhibit the strain** | | |
|---|---|---|
| Strain | - inducer | + inducer |
| RN4220/pYH4 | 0.0625 | 0.0625 |
| RN4220/pYH4-fabI | 0.0625 | 1 |

Figure 4 and Table 1 show the fabI ORF clone confers resistance to triclosan antimicrobial compound when its expression is induced. The resistance to triclosan can be used to select FabI containing strains out of bacterial mixtures, most of which contain non-fabI clones.

In the experiment, a *S. aureus* strain with expression vector (RN4220/pYH4) was combined with the expression vector and FabI open reading frame (RN4220/pYH4-fabI) in the ratio of 100:1. Then, approximately 1,000 bacterial cells of this mixture were plated onto TSA plates containing: (1) no triclosan (-triclosan); (2) 0.25 µg/ml of triclosan; and (3) 0.25 µg/ml of triclosan plus 0.4 µg/ml anhydrotetracycline inducer. About 1000 colonies grew on the -triclosan plate, no colonies grew on the +0.25 µg/ml triclosan plate, and 15 colonies grew on the plate containing 0.25 µg/ml triclosan and 0.4 µg/ml anhydrotetracycline (herein also "Atc") inducer. The third plate was indicative of FabI-containing clones which are confirmed to be targets of the triclosan antimicrobial compound.

As shown in Figure 4, it is possible to identify resistant clones by determining in solution (or on plates) which bacterial clone(s) grows up in the induced population (indicating resistance) when induced and uninduced vectors containing the ORFs of a genome are contacted with a target antimicrobial compound. In the uninduced population, all of the colonies (*i.e.*, ORFs in vectors) will die. In the induced population, a predominant out-growing colony is observed, and that out-growing colony represents the cell carrying a clone with gene targeted by the antimicrobial compound.

### Example 2 Overepression of S. aureus MurA1 confers phosphomycin resistance

Table 2 shows the results of testing whether the murA1 gene is a target of the antimicrobial compound, phosphomycin. The murA1 ORF is cloned into the pYH4 expression vector, the features of which are described above, and the anhydrotetracycline inducer is added to a sample containing the murA1 ORF. The MICs for phosphomycin of induced and uninduced samples are determined and set forth in Table 2. In addition, a control sample containing only the vector is induced and the MICs of the induced and uninduced vector are determined. The MIC of the induced murA1 gene increases significantly and is 64-fold higher than the uninduced vector alone; the MIC of the uninduced murA1 gene increases significantly and is 8-fold higher than the uninduced vector alone. This confirms that murA1 is a target of phosphomycin.

**Table 2**

| **MIC of phosphomycin (µg/ml)** | | |
|---|---|---|
| **Strain** | **-Atc** | **+ATc** |
| RN4220/pYH4 | 4 | 2 |
| RN4220/pYH4*MurA1* | 32 | 256 |
| Note: *murA1* encodes UDP-Nacetylglucosamine enolpyruvyl | | |

### Example 3 Overepression of S. aureus MetrRS confers MetRS inhibitor resistance

Table 3 shows the results of testing whether the metRS gene is a target of an antimicrobial compound believed to be an inhibitor of MetRS, as determined by inhibition of isolated *S. aureus* MetRS protein *in vitro.* The metRS ORF is cloned into the pYH4 expression vector, the features of which are described above, and the anhydrotetracycline inducer is added to a sample containing the metRS ORF. The MICs for the anitmicrobial compound of induced and uninduced samples are determined. In addition, a control sample containing only the vector is induced and the MICs of the induced and uninduced vector are determined. The MIC of the induced metRS gene increases significantly and is 16-fold higher than the uninduced vector alone; the MIC of the uninduced metRS gene increases significantly and is 4-fold higher than the uninduced vector alone. This confirms that metRS is a target of the exemplified antimicrobial compound.

**Table 3**

| **MIC of MetRS Inhibitors (µg/ml)** | | |
|---|---|---|
| **Strain** | **-ATc** | **+ATc** |
| RN4220/pYH4 | 0.25 | 0.25 |
| RN4220/pYH4metRS | 1 | 4 |
| Note: *metRS* encodes methionyl tRNA synthetase (MetRS). | | |

This invention can be used to find the target(s) of compounds which have some antimicrobial activity, but with unknown targets in bacteria. Also, the identity of a suspected target of an antimicrobial compound can be confirmed. Thus, the present invention provides essentially a one-step experiment, which can be carried out overnight, that can identify a cellular or genomic target of an antimicrobial agent.

While this invention has been described with respect to specific examples and embodiments thereof, it is not limited thereto. The claims which follow are intended to be construed to include all modifications of these examples and embodiments, and to such other forms thereof as may be devised by those skilled in the art without departing from the true spirit and scope of the present invention.

All publications and references, including but not limited to patents and patent applications, cited in this specification are herein incorporated by reference in their entirety as if each individual publication or reference were specifically and individually indicated to be incorporated by reference herein as being fully set forth. Any patent application to which this application claims priority is also incorporated by reference herein in its entirety in the manner described above for publications and references.

## Claims

1. A method for identifying a target of an antimicrobial compound comprising:
(a) cloning the open reading frame of a gene of a prokaryotic organism into an expression vector comprising an inducible promoter;
(b) inducing expression of the gene with an inducer in the presence of an antimicrobial compound;
(c) comparing growth of cells from the induced gene expression in the vector to cells from the uninduced gene expression in the vector; and
(d) correlating the comparison to determine if the gene is resistant to the antimicrobial compound and is a target of the compound.

2. The method of claim 1 wherein the growth of cells is compared using the minimum inhibitory concentration of the cells.

3. The method of claim 1 wherein the expression vector is a pYH4 vector having PmeI, NcoI, StuI, and AscI cloning sites.

4. The method of claim 3 wherein the expression vector further comprises a Pxyl/tet regulatory system, a ribosome binding site, and a transcriptional terminator.

5. The method of claim 1 wherein said prokaryotic organism is selected from the group consisting of *Streptococcus, Staphylococcus, Bordetella, Corynebacterium, Mycobacterium, Neisseria, Haemophilus, Actinomycetes, Streptomycetes, Nocardia, Enterobacter, Yersinia, Fancisella, Pasturella, Moraxella, Acinetobacter, Erysipelothrix, Branhamella, Actinobacillus, Streptobacillus, Listeria, alymmatobacterium, Brucella, Bacillus, Clostridium, Treponema, Escherichia, Salmonella, Kleibsiella, Vibrio, Proteus, Erwinia, Borrelia, Leptospira, Spirillum, Campylobacter, Shigella, Legionella, Pseudomonas, Aeromonas, Rickettsia, Chlamydia, Borrelia and Mycoplasma*, and further including, but not limited to, a member of the species or group, *Group A Streptococcus,* *Group B Streptococcus, Group C Streptococcus, Group D Streptococcus, Group G Streptococcus, Streptococcus pneumoniae, Streptococcus pyogenes, Streptococcus agalactiae, Streptococcus faecalis, Streptococcus faecium, Streptococcus durans, Neisseria gonorrheae, Neisseria meningitidis, Staphylococcus aureus, Staphylococcus epidermidis, Corynebacterium diptheriae, Gardnerella vaginalis, Mycobacterium tuberculosis, Mycobacterium bovis, Mycobacterium ulcerans, Mycobacterium leprae, Actinomyctes israelii, Listeria monocytogenes, Bordetella pertusis, Bordatella parapertusis, Bordetella bronchiseptica, Escherichia coli, Shigella dysenteriae, Haemophilus influenzae, Haemophilus aegyptius, Haemophilus parainfluenzae, Haemophilus ducreyi, Bordetella, Salmonella typhi, Citrobacter freundii, Proteus mirabilis, Proteus vulgaris, Yersinia pestis, Kleibsiella pneumoniae, Serratia marcessens, Serratia liquefaciens, Vibrio cholera, Shigella dysenterii, Shigella flexneri, Pseudomonas aeruginosa, Franscisella tularensis, Brucella abortis, Bacillus anthracis, Bacillus cereus, Clostridium perfringens, Clostridium tetani, Clostridium botulinum, Treponema pallidum, Rickettsia rickettsii* and *Chlamydia trachomitis*, (ii) an archaeon, including but not limited to *Archaebacter*, and (iii) a unicellular or filamentous eukaryote, including but not limited to, a protozoan, a fungus, a member of the genus *Saccharomyces, Kluveromyces,* or *Candida,* and a member of the species *Saccharomyces ceriviseae, Kluveromyces lactis*, or *Candida albicans.*

6. A method for detecting a target of an antimicrobial compound comprising:
(a) cloning substantially all of the genes of a prokaryotic organism's genome into expression vectors comprising an inducible promoter to form an ORF expression library;
(b) inducing expression of a copy of said library of genes with an inducer;
(c) contacting the copy of said library of induced genes and a copy of said library of uninduced genes with an antimicrobial compound, the antimicrobial compound killing the cells with non-target genes; and
(d) determining the target gene of the antimicrobial compound by identifying the gene conferring cells survival of the treatment of the antimicrobial compound.

7. The method of claim 6 wherein the genes are fluorescence tagged to determine the identity of the gene conferring resistance to the antimicrobial compound.

8. The method of claim 6 wherein the genes and the antimicrobial compound are plated onto microtiter plates.

9. The method of claim 6 wherein the expressed, surviving gene is contacted with a DNA microarray to determine the identity of the surviving gene resistant to the antimicrobial compound.

10. The method of claim 6 wherein the genes are fluorescence tagged and contacted with a DNA microarray to determine the identity of the surviving gene resistant to the antimicrobial compound.

11. The method of claim 6 wherein the inducer is anhydrotetracycline.

12. The method of claim 6 wherein said prokaryotic organism is selected from the group consisting of *Streptococcus, Staphylococcus, Bordetella, Corynebacterium, Mycobacterium, Neisseria, Haemophilus, Actinomycetes, Streptomycetes, Nocardia, Enterobacter, Yersinia, Fancisella, Pasturella, Moraxella, Acinetobacter, Erysipelothrix, Branhamella, Actinobacillus, Streptobacillus, Listeria, alymmatobacterium, Brucella, Bacillus, Clostridium, Treponema, Escherichia, Salmonella, Kleibsiella, Vibrio, Proteus, Erwinia, Borrelia, Leptospira, Spirillum, Campylobacter, Shigella, Legionella, Pseudomonas, Aeromonas, Rickettsia, Chlamydia, Borrelia and Mycoplasma*, and further including, but not limited to, a member of the species or group, *Group A Streptococcus, Group B Streptococcus, Group C Streptococcus, Group D Streptococcus, Group G Streptococcus, Streptococcus pneumoniae, Streptococcus pyogenes, Streptococcus agalactiae, Streptococcus faecalis, Streptococcus faecium, Streptococcus durans, Neisseria gonorrheae, Neisseria meningitidis, Staphylococcus aureus, Staphylococcus epidermidis, Corynebacterium diptheriae, Gardnerella vaginalis, Mycobacterium tuberculosis, Mycobacterium bovis, Mycobacterium ulcerans, Mycobacterium leprae, Actinomyctes israelii, Listeria monocytogenes, Bordetella pertusis, Bordatella parapertusis, Bordetella bronchiseptica, Escherichia coli, Shigella dysenteriae, Haemophilus influenzae, Haemophilus aegyptius, Haemophilus parainfluenzae, Haemophilus ducreyi, Bordetella, Salmonella typhi, Citrobacter freundii, Proteus mirabilis, Proteus vulgaris, Yersinia pestis, Kleibsiella pneumoniae, Serratia marcessens, Serratia liquefaciens, Vibrio cholera, Shigella dysenterii, Shigella flexneri, Pseudomonas aeruginosa, Franscisella tularensis, Brucella abortis, Bacillus anthracis, Bacillus cereus, Clostridium perfringens, Clostridium tetani,* *Clostridium botulinum, Treponema pallidum, Rickettsia rickettsii* and *Chlamydia trachomitis*, (ii) an archaeon, including but not limited to *Archaebacter*, and (iii) a unicellular or filamentous eukaryote, including but not limited to, a protozoan, a fungus, a member of the genus *Saccharomyces, Kluveromyces,* or *Candida,* and a member of the species *Saccharomyces ceriviseae, Kluveromyces lactis*, or *Candida albicans.*

13. A method of constructing a DNA library of substantially all genes of a prokaryotic organism comprising:
(a) identifying the open reading frames of substantially all genes of a prokaryotic organism;
(b) amplifying the DNA comprising the open reading frames; and
(c) cloning the open reading frames into expression vectors under control of an inducible promoter.

14. The method of claim 13 wherein the DNA is amplified using the polymerase chain reaction.

15. A method of constructing an expression vector comprising incorporating into a DNA vector an inducible promoter system, a ribosome binding site, and multiple cloning sites, said cloning sites allowing the cloning of intact open reading frames of a prokaryotic organism.

16. The method of claim 15 wherein the DNA vector is a pYH4 vector and the cloning sites are PmeI and AscI sites.

17. The method of claim 15 further comprising incorporating into the DNA vector with replicons for *E. coli* and *S. aureus.*
